Europäisches Patentamt

European Patent Office

Office européen des brevets

Numéro de publication: **0 274 928**

**A2**

# DEMANDE DE BREVET EUROPEEN

Numéro de dépôt: 87402703.0

Date de dépôt: 30.11.87

Int. Cl.4 **A61K 9/06** , A61K 31/52 , A61K 31/375 , A61K 31/355 , A61K 31/185 , A61K 31/16

Priorité: 12.12.86 FR 8617430

Date de publication de la demande:
20.07.88 Bulletin 88/29

Etats contractants désignés:
AT BE CH DE FR GB IT LI LU NL SE

Demandeur: **LABORATOIRES CHAUVIN**
**104, Rue de la Galéra**
**F-34009 Montpellier(FR)**

Inventeur: **Bonne, Claude**
**316 avenue d'Occitanie**
**F-34000 Montpellier(FR)**
Inventeur: **Coquelet, Claude**
**113 rue des Lilas**
**F-34980 St. Gely du Fesc(FR)**
Inventeur: **Latour, Elisabeth**
**Les Collines d'Estanove Bat. 2**
**F-34000 Montpellier(FR)**

Mandataire: Le Guen, Gérard et al
**CABINET LAVOIX 2, place d'Estienne d'Orves**
**F-75441 Paris Cédex 09(FR)**

Utilisation d'inhibiteurs de xanthine-oxydase pour la fabrication d'une composition pharmaceutique destinée au traitement du glaucome et compositions pharmaceutiques destinées au traitement du glaucome.

L'invention a pour objet l'utilisation d'un composé choisi parmi les inhibiteurs de xanthine-oxydase pour la fabrication d'une composition pharmaceutique, destinée au traitement du glaucome.

EP 0 274 928 A2

## Utilisation d'inhibiteurs de xanthine-oxydase pour la fabrication d'une composition pharmaceutique destinée au traitement du glaucome et compositions pharmaceutiques destinées au traitement du glaucome

La présente invention concerne le traitement du glaucome et vise plus spécifiquement l'utilisation de certains composés pour la fabrication d'une composition pharmaceutique destinée au traitement du glaucome ainsi que des compositions pharmaceutiques comprenant des associations de composés pour le traitement du glaucome.

Le glaucome primitif à angle ouvert est une maladie oculaire conduisant à la destruction des fibres du nerf optique et à la perte de la vision. Cette destruction est le plus souvent accompagnée d'une hypertension oculaire; toutefois, les lésions ne sont pas directement corrélées à la valeur absolue de cette hypertension.

Les traitements actuels du glaucome consistent à réduire la pression intraoculaire (PIO), mais leur efficacité concernant le maintien de la fonction visuelle n'est pas démontrée.

La Demanderesse a observé que les variations de la PIO chez l'animal induisent, de manière calcium-dépendante, l'apparition d'une activité enzymatique-xanthine-oxydase (forme O) au niveau de nerf optique. Cette enzyme transforme d'hypoxanthine en acide urique sans cofacteur NAD, grâce à l'oxygène moléculaire. La réaction s'accompagne d'une production d'anion superoxyde ($O_2^{\cdot -}$) et de radical $OH^{\cdot}$ par conversion fer-dépendante, provoquant des lésions cellulaires et la destruction des neurones.

La présente invention vise donc à fournir des compositions thérapeutiques capables d'empêcher les lésions induites par ce mécanisme.

La présente invention a ainsi pour objet l'utilisation d'un composé choisi parmi les inhibiteurs de xanthine-oxydase pour la fabrication d'une composition pharmaceutique destinée au traitement du glaucome.

La Demanderesse a en outre découvert que des résultats particulièrement favorables pouvaient être obtenus en utilisant l'association d'un inhibiteur de xanthine-oxydase et d'un piégeur de radicaux libres oxygénés et éventuellement d'un chélateur du fer.

La présente invention a en conséquence également pour objet des compositions pharmaceutiques, pour le traitement du glaucome comprenant l'association d'un inhibiteur de xanthine-oxydase et d'un piégeur de radicaux libres oxygénés et éventuellement d'un chélateur du fer.

Les inhibiteurs de xanthine-oxydase peuvent être notamment l'allopurinol, l'oxypurinol, l'acide folique et des flavonoïdes tels que la myricétine et le kaempférol.

Les piégeurs de radicaux libres oxygénés peuvent être notamment le tocophérol, l'acide ascorbique, les ubiquinones, les sels pharmaceutiquement acceptables de l'acide dihydroxy-2.5 benzène sulfonique (notamment l'éthamsylate), les peroxydases, les dérivés de l'acide caféique et l'anétholtrithione.

Les chélateurs du fer peuvent être notamment la déféroxamine, l'acide éthylènediamino-N,N'-di-(o.hydroxyphénylacétique), la 2,2'-bipyridine, l'acide nitrilotriacétique et l'isonicotinoylhydrazone du pyridoxal.

Une composition particulièrement préférée est celle comprenant
(a) l'association d'allopurinol et d'éthamsylate
Comme exemple d'autres associations on peut citer.
(b) l'association d'allopurinol et de tocophérol et éventuellement d'acide ascorbique
(c) l'association d'acide folique, de tocophérol et d'acide ascorbique
(d) l'association de kaempférol et de tocophérol
(e) l'association de myricétine et d'éthamsylate
(f) l'association d'allopurinol et d'anétholtrithione

Le tableau suivant donne les doses utilisables chez l'homme pour différents principes actifs

| Composé | dose mg/j |
|---|---|
| allopurinol | 100 à 1000 |
| anétholtrithione | 20 à 100 |
| tocophérol | 100 à 1000 |
| acide ascorbique | 300 à 2000 |
| acide folique | 20 à 100 |
| éthamsylate | 300 à 1000 |

Les principes actifs selon l'invention peuvent être administrées à l'homme ou aux animaux par voie topique, orale ou parentérale.

Ces principes actifs peuvent être mis sous forme de compositions pharmaceutiques solides, semi-solides ou liquides. Comme exemples, on peut citer les comprimés, les gélules, les suppositoires, les solutions ou suspensions injectables, ainsi que les formes retard.

Dans ces compositions le principes actif est généralement mélangé avec un ou plusieurs excipients pharmaceutiquement acceptables habituels bien connus de l'homme de l'art.

Les compsitions thérapeutiques administrtables par voie topique peuvent contenir notamment de 0,1 à 5% en poids de principe actif.

Les compositions thérapeutiques administrables par voie orale ou parentérale peuvent contenir notamment de 1 à 60% en poids de principe actif.

Comme exemples des compositions on peut citer les compositions suivantes:

Comprimés- allopurinol      200 mg
- éthamsylate      200 mg
- amidon      60 mg
- Avicel pH 10,2      90 mg
- citrate monosodique   10 mg
- polyvinylpyrolidone   20 mg
- stéarate de magnésium 10 mg

Gélules- allopurinol      200 mg
- acide ascorbique      200 mg
- stéarate de magnésium 10 mg

Soluté buvable- allopurinol      200 mg
- succinate double de
  tocophérol et de PEG   400 mg
- sorbitol      1,5 g
- alcool éthylique 95%   0,2 ml
- composition aromatique qs
- eau      qsp 10 ml

0 274 928

**Revendications**

1. Utilisation d'un composé choisi parmi les inhibiteurs de xanthine-oxydase pour la fabrication d'une composition pharmaceutique, destinée au traitement du glaucome.

2. Utilisation selon la revendication 1, caractérisée en ce que le composé est choisi parmi l'allopurinol, l'oxypurinol, l'acide folique et des flavonoïdes.

3. Utilisation de l'allopurinol pour la fabrication d'un médicament destiné au traitement du glaucome.

4. Composition pharmaceutique pour le traitement du glaucome comprenant l'association d'un inhibiteur de xanthine-oxydase et d'un piégeur de radicaux libres oxygénés et éventuellement d'un chélateur du fer.

5. Composition selon la revendication 4, caractérisée en ce que l'inhibiteur de xanthine-oxydase est choisi parmi l'allopurinol, l'oxypurinol, l'acide folique et des flavonoïdes.

6. Composition selon la revendication 4 ou la revendication 5, caractérisée en ce que le piégeur de radicaux libres oxygénés est choisi parmi le tocophérol, l'acide ascorbique, les ubiquinones, les sels pharmaceutiquement acceptables de l'acide dihydroxy-2,5 benzène sulfonique, les peroxydases, les dérivés de l'acide caféique et l'anétholtrithione.

7. Composition selon l'une quelconque des revendications 4 à 6, caractérisée en ce que le chélateur de fer est choisi parmi la déféroxamine, l'acide éthylène-diamino-N,N'-di(o.hydroxyphénylacétique), la 2,2'-bipyridine, l'acide nitrilotriacétique, l'isonicotinoylhydrazone du pyridoxal.

8. Composition pharmaceutique selon la revendication 4, caractérisée en ce qu'elle comprend une association d'allopurinol et d'éthamsylate.

9. Composition pharmaceutique selon la revendication 4, caractérisée en ce qu'elle comprend une association d'allopurinol et de tocophérol.

4